(19) Europäisches Patentamt
European Patent Office
Office européen des brevets

(11) Veröffentlichungsnummer: **0 229 649**
**B1**

(12) **EUROPÄISCHE PATENTSCHRIFT**

(45) Veröffentlichungstag der Patentschrift: **07.11.90**

(51) Int. Cl.⁵: **C07D 487/04,** A01N 25/32
// (C07D487/04, 239:00, 209:00)

(21) Anmeldenummer: **87100246.5**

(22) Anmeldetag: **10.01.87**

(54) **Optisch aktive Diazabicycloalkanderivate und ihre Verwendung zum Schutz von Kulturpflanzen vor der phytotoxischen Wirkung von Herbiziden.**

(30) Priorität: **15.01.86 DE 3600903**

(43) Veröffentlichungstag der Anmeldung:
**22.07.87 Patentblatt 87/30**

(45) Bekanntmachung des Hinweises auf die Patenterteilung:
**07.11.90 Patentblatt 90/45**

(84) Benannte Vertragsstaaten:
**AT BE CH DE ES FR GB GR IT LI LU NL**

(56) Entgegenhaltungen:
**EP-A- 0 031 042**
**EP-A- 0 065 724**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen(DE)**

(72) Erfinder: **Scholz,Herbert,Dr., Im Finkenschlag 16, D-6730 Neustadt(DE)**
Erfinder: **Zeidler, Adolf, Dr., Ungsteiner Strasse 47, D-6700 Ludwigshafen(DE)**
Erfinder: **Wuerzer, Bruno, Dr., Rüdigerstrasse 13, D-6701 Otterstadt(DE)**
Erfinder: **Meyer,Norbert,Dr., Dossenheimer Weg 22, D-6802 Ladenburg(DE)**

**EP 0 229 649 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft ein optisch aktives Diazabicycloalkanderivat, Mittel zum Schutz von Kulturpflanzen, die dieses Diazabicycloalkanderivat als antagonistisches Mittel und 2-Chlor-6′-ethyl-N-(ethoxymethyl)acet-o-toluidid als herbiziden Stoff enthalten, Verfahren zum Schutz von Kulturpflanzen vor der phytotoxischen Wirkung von 2-Chlor-6′-ethyl-N-(ethoxymethyl)acet-o-toluidid sowie Verfahren zur selektiven Bekämpfung von unerwünschtem Pflanzenwuchs mit den genannten herbiziden Mitteln.

2-Chlor-6′-ethyl-N-(ethoxymethyl)acet-o-toluidid ist herbizid ausgezeichnet wirksam, führt aber bei Anwendung in Kulturen, beispielsweise in Mais, Reis, Kultursorghum oder Getreide zur Schädigung der Kulturpflanze (EP-A 31 402).

Aus der EP-A 31 402 und EP-A 65 724 sind außerdem herbizide Mittel bekannt, die als herbiziden Wirkstoff Acetanilide und als Antagonisten racemische N-Dihalogenacetyldiazabicycloalkanderivate enthalten.

Bei den dort beschriebenen N-Dihalogenacetyldiazabicycloalkanderivaten handelt es sich jeweils um Racemate bzw. Diastereomerengemische. Über die biologische Wirkung der jeweiligen Enantiomeren bzw. Diastereomeren ist nichts offenbart.

Es wurde nun das Diazabicycloalkanderivat der Formel

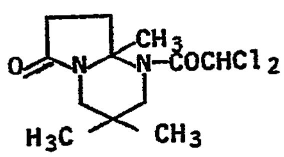

gefunden, bei dem das Kohlenstoffatom mit Brückenkopf-Funktion R-Konfiguration aufweist. Dieses Diazabicycloalkanderivat eignet sich in vorteilhafter Weise zur Steigerung der Kulturpflanzenverträglichkeit von 2-Chlor-6′-ethyl-N-(ethoxymethyl)acet-o-toluidid.

Das erfindungsgemäße Diazabicycloalkanderivat kann durch Umsetzung von S(+)-3,3,6-Trimethyl-9-oxo-1,5-diazabicyclo[4.3.0]nonan, bei dem das Kohlenstoffatom mit Brückenkopf-Funktion S-Konfiguration aufweist, mit Dichloracetylchlorid in Gegenwart eines chlorwasserstoffbindenden Mittels und eines Lösungs- oder Verdünnungsmittels erhalten werden. Die Umesterung wird bei einer Temperatur von –10 und +50°C durchgeführt.

Als Verdünnungs- oder Lösungsmittel kommen gegebenenfalls halogenierte Kohlenwasserstoffe, wie Toluol, Xylole, Chlorbenzol, Dichlormethan oder Ethylenchlorid; Ether, wie Diethylether, Methyltert-butylether, Tetrahydrofuran oder 1,4-Dioxan; oder Nitrile wie Acetonitril, in Betracht.

Geeignete halogenwasserstoffbindende Mittel sind Alkalimetallcarbonate, Alkalimetallhydrogencarbonate, wäßrige Lösungen von Alkalimetallhydroxiden, Trialkylamine, N,N-Dialkylaniline, wie N,N-Dimethylanilin, und Pyridinbasen. Das Amin der Formel II wird zweckmäßig mit der äquimolaren bis 1,2fachen molaren Menge an Dihalogenacetylchlorid umgesetzt. Pro Mol Dihalogenacetylchlorid werden 1 bis 1,2 Mol chlorwasserstoffbindendes Mittel zugegeben.

Das erfindungsgemäße Diazabicycloalkanderivat kann auch durch Umsetzung von S(+)-3,3,6-Trimethyl-9-oxo-1,5-diazabicyclo[4.3.0]nonan mit Chloralhydrat in Gegenwart eines säurebindenden Mittels und katalytischer Mengen Cyanid, das z.B. in Form von Natriumcyanid oder Acetoncyanhydrin zugegeben wird, erhalten werden (DE-A 2 807 340).

Das Racemat bzw. Diastereomerengemisch von 3,3,6-Trimethyl-9-oxo-1,5-diazabicyclo[4.3.0]-nonan, bei dem das Kohlenstoffatom mit Brückenkopf-Funktion sowohl R- und S-Konfiguration aufweist, läßt sich nach dem in DE-A 1 802 468 beschriebenen Herstellungsverfahren durch Umsetzung von γ-Oxo- bzw. δ-Oxocarbonsäuren oder deren Ester mit α,ω-Alkylendiaminen erhalten. Das optisch aktive Amin, bei dem das Kohlenstoffatom mit Brückenkopf-Funktion S-Konfiguration aufweist, kann durch Spaltung des genannten Racemats bzw. Diastereomerengemisches mittels optisch aktiver Säuren hergestellt werden.

Als optisch aktive Säuren verwendet man zweckmäßig chirale Sulfonsäuren, z.B. Camphersulfonsäure oder Bromcamphersulfonsäure sowie chirale Hydroxycarbonsäuren oder deren Derivate, z.B. Milchsäure, Weinsäure, Diacetylweinsäure, Dibenzoylweinsäure, Äpfelsäure oder Mandelsäure. Bevorzugt sind dabei optisch aktive Hydroxycarbonsäuren, wobei Milchsäure und Weinsäure, insbesondere D-(–)-Milchsäure, besonders hervorzuheben ist.

Zur Herstellung der diastereomeren Salze aus racemischem Amin bzw. Amin-Diastereomerengemisch und chiraler Säure gibt man diese in einem geeigneten inerten Lösungsmittel in einem Molverhältnis Amin:Säure von 1:1 bis 1:1,2, vorzugsweise 1:1, bei einer Temperatur von –20 bis +50°C zusammen. Nach einer Nachrührphase von bis zu 5 Stunden, bei der man zweckmäßigerweise eine Temperatur von –20 bis +30°C einhält, kann man das auskristallisierte diastereomere Aminsalz, in dem in der Aminkomponente das Kohlenstoffatom mit Brückenkopf-Funktion S-Konfiguration aufweist, abtrennen.

Geeignete inerte Lösungsmittel für diesen Schritt sind z.B. Alkohole, wie Methanol, Ethanol oder Isopropanol oder Ether, wie 1,2-Dimethoxyethan, tert-Butyl-methylether, Tetrahydrofuran oder 1,4-Dioxan, sowie wäßrige Mischungen dieser Lösungsmittel.

Das abgetrennte diastereomere Aminsalz wird mit Lösungsmittel nachgewaschen, und anschließend wird daraus das gewünschte Amin freigesetzt. Dies geschieht üblicherweise in wäßriger Lösung durch Zusatz von starken Basen, beispielsweise mit wäßrigen Lösungen von Kalium- oder Natriumhydroxid. Besonders vorteilhaft ist es, das gewünschte Amin aus der wäßrigen Lösung des diastereomeren Aminsalzes mittels eines stark basischen Anionenaustauschers freizusetzen.

Das Amin der Formel II fällt dabei üblicherweise in wäßriger Lösung an und kann durch Abdampfen des Wassers unter vermindertem Druck in reiner

Form erhalten und dann direkt mit Dichloracetylchlorid umgesetzt werden.

Es ist überraschend, daß das Amin, bei dem es sich um ein Aminal eines geminalen Amins handelt, unter den Reaktionsbedingungen, d.h. Behandlung mit Säure, keiner Spaltungsreaktion unterliegen, da es bekannt ist, daß solche Aminale durch Säurekatalyse üblicherweise leicht in eine Carbonylverbindung und zwei Aminokomponenten gespalten werden können (P.A.S. Smith, Open-Chain Nitrogen Compounds, Bd. 1, S. 322, W.A. Benjamin, Inc., New York, Amsterdam, 1965). So tritt beispielsweise bei der Trögerschen Base in saurem Milieu Racemisierung ein (E.L. Eliel, Stereochemie der Kohlenstoffverbindungen, S. 466, Verlag Chemie, Weinheim, 1965).

Das bei der Spaltung des Racemats bzw. des Diastereomerengemisches in der Lösung verbleibende Aminsalz, bei dem das Kohlenstoffatom mit Brückenkopf-Funktion die unerwünschte Konfiguration aufweist, kann durch Racemisierung wieder in solche Gemische übergeführt werden, in denen das Kohlenstoffatom mit Brückenkopf-Funktion sowohl R- als auch S-Konfiguration aufweist. Diese Gemische können dann erneut der Spaltung unterworfen werden (Recycling).

Die Racemisierungsreaktion gelingt überraschenderweise ebenfalls sehr vorteilhaft mit Milchsäure (Einsparung von Arbeitsstufen). Dabei wird das Aminsalz, in dem die Aminkomponente die "falsche" Konfiguration aufweist, in wäßriger Lösung auf eine Temperatur von 70 bis 105°C erwärmt. Nach ca. 3 bis 9 Stunden ist die Racemisierung abgeschlossen, und das jeweilige Gemisch kann durch Verdampfen des Wassers in reiner Form erhalten werden.

Dieser Schritt kann sowohl mit dem D(–)- als auch mit dem L(+)-Lactat gleichermaßen durchgeführt werden, wobei die Verwendung des D(–)-Lactats bevorzugt ist.

Es ist natürlich auch möglich, in umgekehrter Weise das Amin zur Trennung von racemischer Milchsäure zu verwenden. Es hat sich gezeigt, daß sowohl (L)- als (D)-3,6,6-Trimethyl-9-oxo-1,5-diazabicyclo[4.3.0]nonan dafür geeignet sind.

Die folgenden Beispiele sollen die Erfindung näher erläutern.

Beispiel 1

3,3,6-Trimethyl-9-oxo-1,5-diazabicyclo[4.3.0]onan-D(–)-lactat

a) 20,5 g (0,113 Mol) racemisches 3,3,6-Trimethyl-9-oxo-1,5-diazabicyclo[4.3.0]nonan werden in 100 ml Tetrahydrofuran vorgelegt. Bei Raumtemperatur tropft man 14 g (0,113 Mol) gereinigte, 75 gew.%ige wäßrige D(–)-Milchsäure zu, rührt 3 Stunden bei Raumtemperatur und bei 10°C nach, saugt ab und wäscht zweimal mit kaltem Tetrahydrofuran.
Ausbeute: 28,6 g
Fp: 70 bis 72°C
$(\alpha)_D^{20}$ = + 3,5°, (c = 5; Wasser) (Die Angabe der Konzentration c erfolgt jeweils in g je 100 ml Lösungsmittel).

b) 2740 g (1 Mol) gereinigte 3,3 gew.%ige wäßrige D(–)-Milchsäure werden vorgelegt. Bei 22°C trägt man langsam 182 g (1 Mol) racemisches 3,3,6-Trimethyl-9-oxo-1,5-diazabicyclo[4.3.0]nonan ein. Die Reaktion ist leicht exotherm, und die Temperatur steigt auf 25°C. Man rührt 1 Stunde nach und engt am Rotationsverdampfer bei einer Badtemperatur von maximal 70°C und bei einem Druck von 30 mm Hg ein. Als Rückstand (277 g) fällt ein rötliches Öl an, zu dem man unter Rühren 300 ml Tetrahydrofuran gibt. Nach ca. 1 Stunde wird die Suspension mit den ausgefallenen farblosen Kristallen auf –5°C abgekühlt, eine halbe Stunde bei –5°C gerührt und mit 3 x 50 ml Tetrahydrofuran (–20°C) gewaschen und im Trockenschrank bei 50°C getrocknet.
Ausbeute: 252,2 g ≙ 92,7%.

Beispiel 2

S(–)-3,3,6-Trimethyl-9-oxo-1,5-diazabicyclo[4.3.0]-nonan-D(–)-lactat

20 g des Lactats aus Beispiel 1 werden in 500 ml Tetrahydrofuran gelöst und unter schwachem Rühren bis auf –20°C abgekühlt. Nach 3 Stunden saugt man den Niederschlag ab.
Ausbeute: 7,2 g
Fp: 103 bis 105°C
$(\alpha)_D^{20}$ = –20,70° (c = 2; Wasser)

Beispiel 3

S(+)-3,3,6-Trimethyl-9-oxo-1,5-diazabicyclo[4.3.0]-nonan

4,7 g (0,017 Mol) des Lactats aus Beispiel 2 werden in 172 ml Wasser gelöst und innerhalb von 3 Stunden über eine Säule (Durchmesser 2 cm, Länge 36 cm), die einen stark basischen Anionenaustauscher enthält, gegeben. Nach Durchlauf der Lösung wird die Säule im gleichen Tempo mit 300 ml Wasser gewaschen. Die gesammelten wäßrigen Lösungen werden im Rotationsverdampfer zur Trockene eingeengt und über Nacht im Trockenschrank getrocknet.
Ausbeute: 3 g = 95,5% d.Th.
Fp: 88,5 bis 89°C
$(\alpha)_D^{20}$ = + 34,2° (c = 2; Wasser)

Beispiel 4

R(–)-5-Dichloracetyl-3,3,6-trimethyl-9-oxo-1,5-diazabicyclo[4.3.0]nonan

3 g (0,01648 Mol) Amin aus Beispiel 3 werden in 16,5 ml Toluol mit 1,9 g (0,0184 Mol) Triethylamin vorgelegt. Bei 30 bis 35°C gibt man innerhalb 15 Minuten 2,7 g (0,0179 Mol) Dichloracetylchlorid zu; man rührt 2 Stunden bei 30°C, gibt 16,5 ml Wasser hinzu und rührt 1 Stunde bei 3°C. Man saugt kalt ab, wäscht zweimal mit 5 ml Wasser und zweimal mit 4 ml kaltem Isopropanol.

Ausbeute: 3,7 g = 77,1% d.Th.
Fp: 170 bis 171°C
$(\alpha)_D^{20}$ = 49° (c = 2; $CHCl_3$)
Gehalt: 99% (HPLC)

Beispiel 5

3,3,6-Trimethyl-9-oxo-1,5-diazabicyclo[4.3.0]-nonan-L(+)-lactat

Durchführung analog Beispiel 1, aber mit L(+)-Milchsäure.
Fp: 71,5 bis 72°C
$(\alpha)_D^{20}$ = 4,2° (c = 5; Wasser)

Beispiel 6

R(+)-3,3,6-Trimethyl-9-oxo-1,5-diazabicyclo[4.3.0]-nonan-L(+)-lactat

Durchführung analog Beispiel 2, aber mit dem L(+)-lactat aus Beispiel 2.
Fp: 103 bis 105,5°C
$(\alpha)_D^{20}$ = + 11,7° (c = 2; Wasser)

Beispiel 7

R(–)-3,3,6-Trimethyl-9-oxo-1,5-diazabicyclo[4.3.0]-nonan

Durchführung analog Beispiel 3, aber mit dem L(+)-Lactat aus Beispiel 6.
Fp: 88,5 bis 89,5°C
$(\alpha)_D^{20}$ = –34,2° (c = 2; Wasser)

Beispiel 8

S(+)-5-Dichloracetyl-3,3,6-Trimethyl-9-oxo-1,5-diazabicyclo[4.3.0]nonan

Durchführung analog Beispiel 4, aber mit dem R(+)-Amin aus Beispiel 7.
Fp: 168 bis 169,5°C
$(\alpha)_D^{20}$ = + 50,9° (c = 2; $CHCl_3$)

Beispiel 9

Racemisierung von R-3,3,6-Trimethyl-9-oxo-1,5-diazabicyclo[4.3.0]nonan-D(–)-lactat

8 g des genannten Lactats $(\alpha)_D^{20}$ = + 12° (c = 2; Wasser) werden in 400 ml Wasser unter Rückfluß erhitzt. Der Drehwert ändert sich im Laufe von 6 Stunden von + 12 auf + 4,5. Nach dem Einengen zur Trockne zeigt der Rückstand einen Schmelzpunkt von 68 bis 72°C.

Der herbizide Wirkstoff und die antagonistisch (antidotisch) als Schutzmittel wirkende Verbindung können gemeinsam oder getrennt nach im Pflanzenbau üblichen Techniken für Pflanzenbehandlungsmittel ausgebracht werden. Sie können z.B. vor oder nach der Saat gemeinsam oder getrennt in den Boden eingearbeitet werden. Das gebräuchlichste Anwendungsverfahren ist die Ausbringung auf die Erdoberfläche unmittelbar nach Ablage der Samen oder in der Zeit zwischen Einsaat und Auflaufen der jungen Pflanzen. Auch eine Behandlung während und nach dem Auflaufen der Kulturpflanzen ist möglich. In jedem Fall kann das antagonistisch wirkende Mittel gleichzeitig mit dem herbiziden Wirkstoff ausgebracht werden. Auch eine getrennte Ausbringung, wobei der Antagonist zuerst und anschließend der herbizide Wirkstoff oder umgekehrt auf das Feld gebracht werden, ist möglich, sofern zwischen Ausbringung beider Substanzen nicht soviel Zeit verstreicht, daß der herbizide Wirkstoff bereits Schäden an den Kulturpflanzen anrichtet. Wirkstoff und Antagonist können hierbei als Spritzmittel in suspendierbarer, emulgierbarer oder löslicher Form oder als Granulate getrennt oder gemeinsam formuliert vorliegen. Eine Behandlung der Kulturpflanzensamen mit dem Antagonisten vor der Aussaat ist ebenfalls möglich. Der herbizide Wirkstoff wird dann allein in der üblichen Weise appliziert.

Abhängig von den jeweils zu behandelnden Kulturen werden, bezogen auf 2-Chlor-6′-ethyl-N-(ethoxymethyl)acet-o-toluidid unterschiedliche Mengen der antagonistisch wirkenden Verbindung benötigt. Die Mengenverhältnisse, in denen 2-Chlor-6′-ethyl-N-(ethoxymethyl)acet-o-toluidid und R(–)-5-Dichloracetyl-3,3,6-trimethyl-9-oxo-1,5-diazabicyclo[4.3.0]nonan eingesetzt werden können, sind über einen weiteren Bereich hinaus variabel. Das Gewichtsverhältnis zwischen 2-Chlor-6′-ethyl-N-(ethoxymethyl)acet-o-toluidid und R-(–)-5-Dichloracetyl-3,3,6-trimethyl-9-oxo-1,5-diazabicyclo-[4.3.0]nonan beträgt erfindungsgemäß 1:2 bis 1:0,001, vorzugsweise 1:0,25 bis 1:0,005 und insbesondere 1:0,01.

Die erfindungsgemäßen 2-Chlor-6′-ethyl-N-(ethoxymethyl)acet-o-toluidid und R(–)-5-Dichloracetyl-3,3,6-trimethyl-9-oxo-1,5-diazabicyclo-[4.3.0]nonan enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser, in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol oder Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetat, Sorbitester, Lignin, Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.% an 2-Chlor-6'-ethyl-N-(ethoxymethyl)-acet-o-toluidid und R(–)-5-Dichloracetyl-3,3,6-trimethyl-9-oxo-1,5-diazabicyclo[4.3.0]nonan oder R(–)-5-Dichloracetyl-3,3,6-trimethyl-9-oxo-1,5-diazabicyclo[4.3.0]nonan allein, vorzugsweise zwischen 0,5 und 90 Gew.%. Die Aufwandmengen an 2-Chlor-6'-ethyl-N-(ethoxymethyl)acet-o-toluidid betragen zwischen 0,1 bis 0,5 kg/ha. Diese Menge an 2-Chlor-6'-ethyl-N-(ethoxymethyl)acet-o-toluidid wird, gemeinsam oder getrennt, mit einer solchen Menge an R(–)-5-Dichloracetyl-3,3,6-trimethyl-9-oxo-1,5-diazabicyclo[4.3.0]nonan ausgebracht, daß das Gewichtsverhältnis 2-Chlor-6'-ethyl-N-(ethoxymethyl)acet-o-toluidid : R(–)-5-Dichloracetyl-3,3,6-trimethyl-9-oxo-1,5-diazabicyclo[4.3.0]-nonan, wie oben bereits genannt, 1:2 bis 1:0,001, vorzugsweise 1:0,25 bis 1:0,005 und insbesondere 1:0,25 bis 1:0,01 beträgt.

Beispiele für Formulierungen sind:

I. 40 Gew.-Teile der Mischung aus 4 Gew.-Teilen 2-Chlor-2',6'-dimethyl-N-(pyrazol-1-yl-methyl)-acetanilid und 1 Gew.-Teil R(-)-5-Dichloracetyl-3,6,6-trimethyl-9-oxo-1,5-diazabicyclo[4.3.0]nonan werden mit 10 Gew.-Teilen Natriumsalz eines Phenolsulfonsäure-Harnstoff-Formaldehyd-Kondensats, 2 Gew.-Teilen Kieselgel und 48 Teilen Wasser innig vermischt. Man erhält eine stabile wäßrige Dispersion. Durch Verdünnen mit 100.000 Gew.-Teilen Wasser erhält man eine wäßrige Dispersion, die 0,04 Gew.% Wirkstoff enthält.

II. 3 Gew.-Teile der Mischung aus 1 Gew.-Teil 2-Chlor-2'-methyl-6'-ethyl-N-(pyrazol-1-yl-methyl)-acetanilid und 1 Gew.-Teil R(-)-5-Dichloracetyl-3,6,6-trimethyl-9-oxo-1,5-diazabicyclo[4.3.0]nonan werden mit 97 Gew.-Teilen feinteiligem Kaolin innig vermischt. Man erhält auf diese Weise ein Stäubemittel, das 3 Gew.% des Wirkstoffs enthält.

III. 30 Gew.-Teile der Mischung aus 1 Gew.-Teil 2-Chlor-2'-methyl-6'-ethyl-N-(1,2,4-triazol-1-yl-methyl)-acetanilid und 2 Gew.-Teilen R(-)-5-Dichloracetyl-3,6,6-trimethyl-9-oxo-1,5-diazabicyclo-[4.3.0]nonan werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

IV. 20 Gew.-Teile der Mischung aus 8 Gew.-Teilen 2-Chlor-2'-methyl-6'-ethyl-N-ethoxymethyl-acetanilid und 1 Gew.-Teil R(-)-5-Dichloracetyl-3,6,6-trimethyl-9-oxo-1,5-diazabicyclo[4.3.0]nonan werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-Harnstoff-Formaldehyd-Kondensats und 68 Gew.-Teilen eines paraffinischen Mineralöls innig vermischt. Man erhält eine stabile ölige Dispersion.

V. 20 Gew.-Teile der Mischung aus 10 Gew.-Teilen 2-Chlor-2',6'-dimethyl-N-(2-methoxyethyl)-acetanilid und 1 Gew.-Teil R(-)-5-Dichloracetyl-3,6,6-trimethyl-9-oxo-1,5-diazabicyclo[4.3.0]nonan werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100.000 Gew.-Teilen Wasser erhält man eine wäßrige Dispersion, die 0,02 Gew.% des Wirkstoffs enthält.

Die neuen herbiziden Mittel können neben 2-Chlor-6'-ethyl-N-(ethoxymethyl)-acet-o-toluidid und R(–)-5-Dichloracetyl-3,3,6-trimethyl-9-oxo-1,5-diazabicyclo[4.3.0]nonan weitere herbizide oder wachstumsregulierende Wirkstoffe anderer chemischer Struktur enthalten, wobei der antagonistische Effekt erhalten bleibt. Sie können beispielsweise 2-Chlor-4-ethylamino-6-isopropylamino-1,3,5-triazin, 2-(2-Chlor-4-ethylamino-1,3,5-triazin-6-yl-amino)-2-methyl-propionitril, N-(1-Ethyl-n-propyl)-2,6-dinitro-3,4-dimethylanilin enthalten.

## Patentansprüche

1. Optisch aktives Diazabicycloalkanderivat der Formel

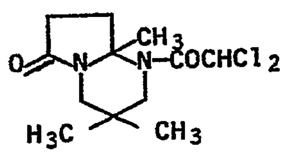

wobei das Kohlenstoffatom mit Brückenkopf-Funktion R-Konfiguration aufweist.

2. Herbizide Mittel, gekennzeichnet durch einen Gehalt an 2-Chlor-6′-ethyl-N-(ethoxymethyl)-acet-o-toluidid als herbizidem Wirkstoff und dem optisch aktiven Diazabicycloalkanderivat gemäß Anspruch 1 als antigonistisches Mittel.

3. Herbizides Mittel gemäß Anspruch 4, dadurch gekennzeichnet, daß das Gewichtsverhältnis optisch aktives Diazabicycloalkanderivat: 2-Chlor-6′-ethyl-N-(ethoxymethyl)-acet-o-toluidid bei gemeinsamer oder getrennter Ausbringung 2:1 bis 0,001:1 beträgt.

4. Verfahren zum Schutz von Kulturpflanzen vor der phytotoxischen Wirkung von 2-Chlor-6′-ethyl-N-(ethoxymethyl-acet-o-toluidid, dadurch gekennzeichnet, daß man die Kulturpflanze, deren Samen oder deren Standort mit einer wirksamen Menge des optisch aktiven Diazabicycloalkanderivats gemäß Anspruch 1 behandelt.

5. Verfahren zur selektiven Bekämpfung von unerwünschtem Pflanzenwuchs, dadurch gekennzeichnet, daß man 2-Chlor-6′-ethyl-N-(ethoxymethyl)-acet-o-toluidid und das optisch aktive Diazabicycloalkanderivat gemäß Anspruch 1 vor, während oder nach der Aussaat der Kulturpflanzen oder vor oder während des Auflaufens der Kulturpflanzen gleichzeitig oder nacheinander in beliebiger Reihenfolge ausbringt.

6. Verfahren zur selektiven Bekämpfung von unerwünschtem Pflanzenwuchs mit 2-Chlor-6′-ethyl-N-(ethoxymethyl)-acet-o-toluidid, dadurch gekennzeichnet, daß man das Saatgut der Kulturpflanzen mit dem optisch aktiven Diazabicycloalkanderivat gemäß Anspruch 1 behandelt.

## Claims

1. An optically active diazabicycloalkane derivative of the formula

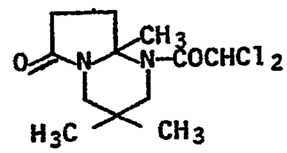

where the carbon atom functioning as bridgehead has the R configuration.

2. A herbicidal agent which contains 2-chloro-6'ethyl-N-(ethoxmethyl)-acet-o-toluidide as herbicidal active ingredient and an optically active diazabicycloalkane derivative as claimed in claim 1 as antagonistic agent.

3. A herbicidal agent as claimed in claim 2, wherein the ratio of optically active diazabicycloalkane derivative to 2-chloro-6'-ethyl-N-(ethoxymethyl)-acet-o-toluidide in the case of joint or separate application is from 2:1 to 0.001:1 by weight.

4. A process for protecting crop plants against the phytotoxic action of 2-chloro-6'-ethyl-N-(ethoxymethyl)acet-o-toluidide, wherein the crop plant, its seed or its location is treated with an effective amount of an optically active diazabicycloalkane derivative as claimed in claim 1.

5. A process for the selective control of unwanted plant growth, wherein 2-chloro-6'-ethyl-N-(ethoxymethyl)-acet-o-toluidide and an optically active diazabicycloalkane derivative as claimed in claim 1 are applied simultaneously or one after the other in any order before, during or after sowing of the crop plants or before or during emergence of the crop plants.

6. A process for the selective control of unwanted plant growth with 2-chloro-6'-ethyl-N-(ethoxymethyl)acet-o-toluidide, wherein the seed of the crop plants is treated with an optically active diazabicycloalkane derivative as claimed in claim 1.

## Revendications

1. Dérivé de diazabicycloalcane optiquement actif de la formule

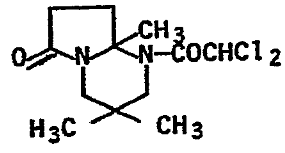

l'atome de carbone ayant une configuration R à fonction de tête de pont.

2. Agent herbicide, caractérisé par une teneur en 2-chloro-6′-éthyl-N-(éthoxyméthyl)-acét-o-toluide comme principe actif herbicide et en le dérivé de diazabicycloalcane optiquement actif selon la revendication 1 comme agent antagoniste.

3. Agent herbicide selon la revendication 2, caractérisé par le fait que le rapport en poids du dérivé de diazabicycloalcane optiquement actif au 2-chloro-6'-éthyl-N-(éthoxyméthyl)-acét-o-toluide est, par application commune ou séparée, de 2/1 à 0,001/1.

4. Procédé pour la protection de plantes cultivées contre l'action phytotoxique du 2-chloro-6'-éthyl-N-(éthoxyméthyl)-acét-o-toluide, caractérisé par le fait qu'on traite les plantes cultivées, leurs semences ou leur habitat par une quantité efficace du dérivé de diazabicycloalcane optiquement actif selon la revendication 1.

5. Procédé pour lutter sélectivement contre la croissance de plantes indésirables, caractérisé par le fait qu'on applique du 2-chloro-6'-éthyl-N-(éthoxyméthyl)-acét-o-toluide et le dérivé de diazabicycloalcane optiquement actif selon la revendication 1 en même temps ou successivement dans un ordre quelconque, avant, pendant ou après le semis des plantes de culture ou avant ou pendant l'émergence des plantes de culture.

6. Procédé pour lutter sélectivement contre la croissance de plantes indésirables, avec du 2-chloro-6'-éthyl-N-(éthoxyméthyl)-acét-o-toluide, caractérisé par le fait qu'on traite la semence des plantes de culture avec le dérivé de diazabicycloalcane optiquement actif selon la revendication 1.